# EUROPÄISCHE PATENTSCHRIFT

(11) **EP 4 553 484 B1**
(45) Veröffentlichungstag und Bekanntmachung des Hinweises auf die Patenterteilung: **25.11.2026**
(21) Anmeldenummer: 24203062.5
(22) Anmeldetag: 26.09.2024
(51) Int. Cl.: G01N 11/08, G01N 11/00

(54) **INLINE-VISKOSITÄTSMESSVERFAHREN, VERWENDUNG SOWIE EINE ZUGEHÖRIGE INLINE-VISKOSITÄTSMESSVORRICHTUNG**
INLINE VISCOSITY MEASUREMENT METHOD, USE AND CORRESPONDING INLINE VISCOSITY MEASUREMENT DEVICE
PROCÉDÉ DE MESURE DE LA VISCOSITÉ EN LIGNE, UTILISATION ET DISPOSITIF DE MESURE DE LA VISCOSITÉ EN LIGNE ASSOCIÉ

(30) Priorität: 07.11.2023 EP 23208410
(43) Veröffentlichungstag der Anmeldung: 14.05.2025
(73) Patentinhaber: Promix Solutions AG, 8406 Winterthur (CH)
(72) Erfinder: Heusser, Rolf, 8400 Winterthur (CH)
(74) Vertreter: Herrmann, Johanna

(56) Entgegenhaltungen:
- CN-U- 215 262 978
- DE-A1- 3 306 476
- DE-A1- 4 001 341
- FR-A- 1 228 750
- US-A1- 2003 226 405
- US-A1- 2008 134 765

## Beschreibung

### Hintergrund

Die Erfindung betrifft ein Inline-Viskositätsmessverfahren sowie eine zugehörige Inline-Viskositätsmessvorrichtung zur Messung einer Druckdifferenz zur Bestimmung einer Viskosität eines fliessfähigen Mediums. Die Erfindung betrifft insbesondere die Verwendung des Verfahrens in einem Verarbeitungsprozess für PVC. sowie eine Vorrichtung zur Messung einer Druckdifferenz für viskose Flüssigkeiten oder Slurries, beispielsweise für PVC, insbesondere ein Viskosimeter.

In einem PVC-Verarbeitungsverfahren ist die Viskosität der PVC-Schmelze eine interessante Kennzahl, die Rückschlüsse auf die Rohstoffzusammensetzung und die Prozessparameter zulässt. Daher ist es vorteilhaft, die Viskosität kontinuierlich und prozessbegleitend zu messen. Die kontinuierliche Messung der Viskosität ermöglicht es, während des Verarbeitungsprozesses einzugreifen, um bei Bedarf Rohstoffzusammensetzung oder Prozessparameter anzupassen.

Die Herstellung von PVC erfolgt in der Regel in einem Extrusionsverfahren. PVC wird in der Regel als Feststoff in Pulverform oder als Granulat als Ausgangsmaterial für das Extrusionsverfahren verwendet. Das PVC-Pulver oder PVC-Granulat wird dem Extruder zugeführt und unter Zufuhr von Wärme teilweise zu einer PVC-Schmelze aufgeschmolzen. Die PVC-Schmelze, welche den Extruder verlässt, wird einem formgebenden Werkzeug zugeführt, in welchem die PVC-Schmelze zu einem gewünschten Produkt geformt wird. Das Produkt, welches das formgebende Werkzeug verlässt, wird im Anschluss bis zur Erstarrung der PVC-Schmelze abgekühlt. Ein derartiges Produkt kann beispielsweise eine Folie, ein Profil, ein Rohrelement, ein Kabel oder auch ein Granulat sein.

### Stand der Technik

Bei PVC tritt im fliessfähigen Zustand das sogenannte Wandgleiten auf. Beim Wandgleiten handelt es sich um ein rheologisches Phänomen, welches nur bei einer geringen Anzahl an Polymeren beobachtet wird, unter anderem PVC. Unter gewissen Prozessbedingungen entsteht eine dünne Schicht in der PVC-Schmelze in der Nähe einer Kanalwandung eines Kanals durch welchen die PVC-Schmelze strömt, welche eine niedrigere Viskosität aufweist als die PVC-Schmelze ausserhalb der dünnen Schicht, also insbesondere der PVC-Schmelze, die in grösserer Entfernung von der Kanalwand strömt. Die Wandreibung kann durch die niedrigere Viskosität der dünnen Schicht reduziert sein. Daher kann ein Messgerät, wie beispielsweise in der US2005/0178442 A1 beschrieben, welches eine Messung in der dünnen Schicht ausführt, bedingt durch die Wandreibung der Kanalwand, einen fehlerhaften Wert für die Viskosität ermitteln. Denn dieses Messgerät ermittelt die Viskosität der dünnen Schicht, sodass der Messwert durch die dünne Schicht stark beeinflusst wird. Eine solche Messung ist nicht repräsentativ für die Viskosität der PVC-Schmelze ausserhalb der dünnen Schicht. Messgeräte, die an der Kanalwand angebracht sind, oder Messgeräte, die eine Verengung eines Kanals zur Messung verwenden, wie beispielsweise eine Kapillare gemäss DE 40 01 341 A1, oder wie beispielsweise Blenden, ermitteln einen Druckverlust über die Wandreibung der Kanalwand. Durch das Wandgleiten wird ein zu niedriger Druckverlust gemessen und somit eine zu niedrige Viskosität ermittelt.

Aus diesen Gründen können mit handelsüblichen Inline-Viskositätsmessgeräten für PVC-Schmelzen und ähnliche fliessfähige Medien, in welchen das Wandgleiten auftritt, keine zuverlässigen und reproduzierbaren Viskositätsmesswerte erzeugt werden. Die Messung des Druckverlustes über eine Blende eignet sich somit nicht für die Ermittlung der Viskosität von PVC-Schmelzen, da die Wandgleiteigenschaften eine reproduzierbare und aussagekräftige Messung des Druckverlustes verhindern. Der Druckverlust liefert die Basiskenngrösse für die Ermittlung der Viskosität. Mit einer nicht reproduzierbaren Basiskenngrösse kann folglich in der Praxis kein zufriedenstellender Messwert für die Viskosität ermittelt werden. Da das Wandgleiten zudem von verschiedenen Faktoren, wie zum Beispiel Rohstoffzusammensetzung, Prozesstemperatur und Scherverhalten abhängt, kann die über eine herkömmliche Blende gemessene Viskosität auch nicht einfach mit einem Faktor korrigiert werden.

Zudem wird PVC meistens nicht als reine PVC-Schmelze verarbeitet. Je nach Geliergrad sind in der PVC-Schmelze noch nicht geschmolzene Feststoffe enthalten, welche die Viskosität zusätzlich beeinflussen. Bei einer Messung des Druckverlustes über eine Blende wird der Einfluss der Feststoffe auf die Viskosität nicht erfasst, da das Wandgleiten die Messung überlagert. Ohne Wandgleiten wäre die Zusammensetzung der Schmelze an oder in der Nähe der Kanalwand gleich wie im Innenbereich der Schmelze, sodass die nicht geschmolzenen Feststoffe sich auf das Messergebnis auswirken würden und somit ein der Zusammensetzung der Schmelze entsprechendes korrektes Messergebnis erhältlich wäre. Mit dem Innenbereich ist der Bereich der Schmelze gemeint, der genügenden Abstand von der Kanalwand aufweist. Aus diesen Gründen können mit herkömmlichen Druckverlustmessungen über Blenden und Rohrverengungen keine repräsentativen und reproduzierbaren Viskositäten berechnet werden.

Eine andere Herausforderung bei der Verarbeitung von PVC liegt im hohen Risiko der Produktzersetzung. Herkömmliche Inlineviskosimeter mit Bypass, wie beispielsweise im Dokument DE 3306476 gezeigt, oder die Verwendung einer Schmelzepumpe erfordern eine zu hohe Verweilzeit der PVC-Schmelze in diesen Messgeräten, was zu einer Zersetzung desselben führt und somit ebenfalls zu irreführenden Messergebnissen führen kann.

Aufgrund der Wandgleiteigenschaften kann sich die Reibung zwischen der Kanalwandung und der PVC-Schmelze je nach Prozessbedingungen schlagartig verändern, insbesondere kann sich die Reibung schlagartig verringern. Daher hat sich herausgestellt, dass mit herkömmlichen Inlineviskosimetern für PVC-Schmelzen, insbesondere Hart-PVC enthaltende Schmelzen, keine reproduzierbaren Messergebnisse generiert werden können, insbesondere mit Inlineviskosimetern, die Blenden oder Kanalverengungen enthalten, über welche der Druckverlust gemessen wird und daraus die Viskosität berechnet wird.

Gemäss CN215262978U wird ein Messgerät für einen Viskositätskoeffizienten vorgeschlagen, welches ein Widerstandsmessrohr enthält. Das Widerstandsmessrohr enthält eine Strömungsstabilisierungsvorrichtung auf der linken und rechten Seite des Widerstandsmessrohrs sowie eine oben mittig angeordnete Widerstandsmessvorrichtung, die als dünner Stab mit einem kugelförmigen Ende ausgebildet ist. Der dünne Stab ist an der oberen Innenwand des Widerstandsmessrohrs befestigt und ragt in das durch das Widerstandsmessrohr fliessende viskose Fluid hinein. Der dünne Stab mit dem kugelförmigen Ende wird durch das Fluid ausgelenkt. Die Auslenkung wird durch die Widerstandsmessvorrichtung gemessen. Zusätzlich wird die Druckdifferenz über zwei mit dem Widerstandsmessrohr verbundene Nebenleitungen gemessen, die stromabwärts und stromaufwärts des Widerstandsmessgeräts angeordnet sind. Die Widerstandsmessvorrichtung und der dünne Stab mit dem kugelförmigen Ende werden anschliessend entfernt, um den Rohrleitungswiderstandskoeffizienten zu ermitteln. Dieses Messgerät eignet sich nicht für eine Inlinemessung, da der erforderliche Unterbruch der Messtätigkeit dazu führen würde, dass für eine Charge, die während des Unterbruchs der Messtätigkeit verarbeitet würde, keine entsprechenden Viskositätsdaten zur Verfügung stehen.

Dokument US 2008/134765 A1 zeigt ein Inlineviskosimeter für eine Mikroprobe eines fliessfähigen Materials. Dieses Viskosimeter enthält einen Mikrokanal mit einem Mikroschlitz mit integrierten Drucksensoren. Der Mikrokanal hat eine Kanalhöhe von 1 Mikrometer. Die Kanalbreite beträgt mehr als das Zehnfache der Kanalhöhe. Nach einem Ausführungsbeispiel beträgt die Kanalhöhe 100 Mikrometer. Im Kanal befindet sich ein Schlitz, der durch Vorsprünge ausgebildet wird. Dieses Inlineviskosimeter eignet sich nicht für fliessfähige Medien, in denen ein Wandgleiten auftritt. Es ist davon auszugehen, dass in einem Kanal mit einer Kanalhöhe von nur einem Mikrometer die Viskosität ausschliesslich für den Bereich eines Strömungskanals ermittelt werden kann, in welchem Wandgleiten auftritt. Dieses Inlineviskosimeter eignet sich aufgrund der angegebenen Abmessungen des Strömungskanals nicht für die Messung einer Kernströmung.

### Aufgabe der Erfindung

Aufgabe der Erfindung ist es, ein zuverlässiges und kostengünstiges Verfahren zu entwickeln, um Druckdifferenzen von fliessfähigen Medien, die zum Wandgleiten neigen, insbesondere PVC -Schmelzen, direkt im Verarbeitungsprozess, das heisst inline, zu messen, beispielsweise zur Bestimmung einer Viskosität.

Insbesondere ist es Aufgabe der Erfindung, die Viskosität von Hart-PVC im Verarbeitungsprozess, wie zum Beispiel einem Extrusionsverfahren, inline, kontinuierlich, zuverlässig und kostengünstig zu messen.

### Beschreibung der Erfindung

Die Lösung der Aufgabe der Erfindung erfolgt durch ein Inline-Viskositätsmessverfahren gemäss Anspruch 1. Vorteilhafte Verfahrensvarianten sind Gegenstand der Ansprüche 2 bis 7. Anspruch 8 bezieht sich auf die Verwendung des Inline-Viskositätsmessverfahrens in einem Verarbeitungsprozess für PVC. Ansprüche 9 bis 12 beziehen sich auf vorteilhafte Verwendungsvarianten für das Inline-Viskositätsmessverfahren. Eine Inline-Viskositätsmessvorrichtung, welche zur Durchführung des erfindungsgemässen Inline-Viskositätsmessverfahrens geeignet ist, ist Gegenstand des Anspruchs 13.

Wenn der Begriff "beispielsweise" in der nachfolgenden Beschreibung verwendet wird, bezieht sich dieser Begriff auf Ausführungsbeispiele und/oder Ausführungsformen, was nicht notwendigerweise als eine bevorzugtere Anwendung der Lehre der Erfindung zu verstehen ist. In ähnlicher Weise sind die Begriffe "vorzugsweise", "bevorzugt" zu verstehen, indem sie sich auf ein Beispiel aus einer Menge von Ausführungsbeispielen und/oder Ausführungsformen beziehen, was nicht notwendigerweise als eine bevorzugte Anwendung der Lehre der Erfindung zu verstehen ist. Dementsprechend können sich die Begriffe "beispielsweise", "vorzugsweise" oder "bevorzugt" auf eine Mehrzahl von Ausführungsbeispielen und/oder Ausführungsformen beziehen.

Die nachfolgende detaillierte Beschreibung enthält verschiedene Ausführungsbeispiele für das erfindungsgemässe Inline-Viskositätsmessverfahren sowie die erfindungsgemässe Inline-Viskositätsmessvorrichtung. Die Beschreibung eines bestimmten Verfahrens oder einer bestimmten Vorrichtung ist nur als beispielhaft anzusehen. In der Beschreibung und den Ansprüchen werden die Begriffe "enthalten", "umfassen", "aufweisen" als "enthalten, aber nicht beschränkt auf" interpretiert.

Ein Inline-Viskositätsmessverfahren zur Messung einer Druckdifferenz zur Bestimmung einer Viskosität eines fliessfähigen Mediums umfasst die nachfolgenden Schritte: das fliessfähige Medium durchströmt eine in einem Rohrelement angeordnete Messstrecke, wobei die Messstrecke als ein Strömungskanal ausgebildet ist, wobei das Rohrelement eine Längsachse, ein Eintrittsende und ein Austrittsende umfasst. Die Messstrecke erstreckt sich zwischen dem Eintrittsende und dem Austrittsende. In der Messstrecke ist zumindest ein Einbauelement angeordnet. Ein Eintrittsdruck wird am Eintrittsende von einem Eintrittsdrucksensor gemessen, sodass ein Eintrittsdruckmesswert erhalten wird und ein Austrittsdruck wird am Austrittsende gemessen, sodass ein Austrittsdruckmesswert erhalten wird. Gemäss eines Ausführungsbeispiels wird der Austrittsdruck mittels eines Austrittsdrucksensors ermittelt. Alternativ kann als Austrittsdruck auch der Umgebungsdruck verwendet werden. Gemäss dieses Ausführungsbeispiels erstreckt sich die Messstrecke bis zum Austrittsende eines formgebenden Werkzeugs, welches stromabwärts des Rohrelements angeordnet ist. Der Eintrittsdruckmesswert und der Austrittsdruckmesswert werden mittels eines Wandlers in von einer Rechnereinheit verarbeitbare Messgrössen umgewandelt. Die Rechnereinheit ermittelt einen Differenzdruck zwischen dem Eintrittsdruckmesswert und dem Austrittsdruckmesswert. Das Einbauelement ist als zumindest ein Stegelement ausgebildet, wobei das Stegelement mit einer Stegelementlänge LS in den Strömungskanal hineinragt, die mindestens 25% eines Durchmessers DS des Strömungskanals beträgt.

Insbesondere weist das Stegelement eine Stegelementbreite auf, die maximal 30% der Stegelementlänge LS beträgt. Das Stegelement ragt somit fingerartig in das im Strömungskanal fliessende fliessfähige Medium.

Nach einem Ausführungsbeispiel weist das Stegelement ein erstes Stegelementende und ein zweites Stegelementende auf, wobei weder das erste Stegelementende noch das zweite Stegelementende mit dem Rohrelement verbunden sind. Insbesondere weisen sowohl das erste Stegelementende als auch das zweite Stegelementende einen Abstand von einer Innenwand des Rohrelements auf, der mindestens 10% des Innendurchmessers des Rohrelements entspricht. Nach einem Ausführungsbeispiel enthält das Stegelement einen Stegelementarm, welcher als ein Verbindungselement mit der Innenwand des Rohrelements ausgebildet ist.

Nach einem Ausführungsbeispiel sind mindestens 60% der Querschnittsfläche des Rohrelements durch das oder die Stegelemente abgedeckt. Insbesondere sind mindestens 60% der Querschnittsfläche und maximal 90% der Querschnittsfläche des Rohrelements durch das oder die Stegelemente abgedeckt. Insbesondere können mindestens zwei Stegelemente vorgesehen sein. Wenn zwei oder mehr Stegelemente vorgesehen sind, können sie miteinander verbunden sein.

Ein Durchflussmesswert des durch das Rohrelement strömenden fliessfähigen Mediums kann beispielsweise mittels eines Durchflusssensors ermittelt werden oder ein mit dem Durchfluss korrelierter Messwert kann beispielsweise über die Drehzahl eines Schneckenelements eines Extruders ermittelt werden. Ein Temperatursensor kann einen Temperaturmesswert des durch das Rohrelement strömenden fliessfähigen Mediums ermitteln. Die Rechnereinheit kann aus dem Differenzdruck und einem Durchflussmesswert eine Viskosität ermitteln. Das Einbauelement ist als zumindest eine Gruppe von Stegelementen ausgebildet. Die Gruppe von Stegelementen enthält vorzugsweise eine Mehrzahl von Stegelementen.

Die Gruppe von Stegelementen enthält gemäss eines Ausführungsbeispiels mindestens zwei Stegelemente. Gemäss eines Ausführungsbeispiels enthält die Gruppe von Stegelementen mindestens vier Stegelemente. Die Stegelemente der Gruppe von Stegelementen können dadurch gekennzeichnet sein, dass sie parallel zueinander angeordnet sind. Die Stegelemente erstrecken sich insbesondere von einer Innenwand des Rohrelements in den Innenraum des Rohrelements. Die Länge der Stegelemente beträgt insbesondere mindestens ein Viertel, vorzugsweise mindestens ein Drittel des Innendurchmessers des Rohrelements.

Der Innendurchmesser des Rohrelements entspricht dem Durchmesser DS des Strömungskanals. Der Innendurchmesser eines nicht kreisförmigen Rohrelements wird als äquivalenter Innendurchmesser gemäss der in der Folge angegebenen Berechnungsvorschrift ermittelt. Die Berechnungsvorschrift gilt sinngemäss auch für den Durchmesser DS des Strömungskanals.

Es hat sich gezeigt, dass der Druckverlust als Basis für die Bestimmung der Viskosität reproduzierbar und aussagekräftig über derartige, in das fliessfähige Medium eingreifende Stegelemente gemessen werden kann. Dabei hat sich gezeigt, dass Wandgleiten bei den eingreifenden Stegelementen nicht auftritt oder zumindest keinen störenden Einfluss auf das Messergebnis hat und sich reproduzierbare Druckverluste messen lassen, die als Basis für die Ermittlung der Viskosität verwendet werden können.

Wenn das fliessfähige Medium Feststoffe enthält, können auch Einflüsse, die durch diese Feststoffe bedingt sind, mit erfasst werden.

Die Stegelemente erstrecken sich in einer ersten Gruppenebene und einer zweiten Gruppenebene, wobei die erste Gruppenebene einen ersten Winkel mit der Längsachse des Rohrelements einschliesst und die zweite Gruppenebene einen zweiten Winkel mit der Längsachse des Rohrelements einschliesst. Gemäss einer Verfahrensvariante kann jede der Gruppenebenen mindestens zwei Stegelemente enthalten. Die Stegelemente jeder der Gruppenebenen können dadurch gekennzeichnet sein, dass sie parallel zueinander angeordnet sind. Gemäss einer Verfahrensvariante kreuzt sich die erste Gruppenebene mit der zweiten Gruppenebene.

Gemäss einer Verfahrensvariante weist zumindest einer des ersten Winkels und des zweiten Winkels gemessen in Bezug auf die Längsachse einen Wert von ungleich 90 Grad auf. Damit die störenden Wandgleiteigenschaften möglichst wenig in Erscheinung treten, werden somit Stegelemente verwendet, die in einem Winkel ungleich 90 Grad zur Strömungsrichtung angebracht sind. Die Strömungsrichtung entspricht der Längsachse des Rohrelements.

Gemäss einer Verfahrensvariante enthalten die erste Gruppenebene und die zweite Gruppenebene mindestens je ein Stegelement. Gemäss einer Verfahrensvariante enthalten zumindest eine der ersten und der zweiten Gruppenebenen zumindest zwei Stegelemente. Insbesondere enthält jede der ersten und zweiten Gruppenebenen mindestens zwei Stegelemente. Gemäss einer Verfahrensvariante sind eine Mehrzahl von Gruppen von Stegelementen in der Messstrecke hintereinander angeordnet.

Gemäss einer Verfahrensvariante weist das Rohrelement einen Innendurchmesser auf, wobei zumindest eines der Stegelemente eine Stegelementlänge LS aufweist, die grösser als der Innendurchmesser ist. Gemäss einer Verfahrensvariante beträgt die Stegelementlänge LS maximal das Dreifache des Innendurchmessers des Rohrelements. Zudem hat es sich gezeigt, dass die Stegelemente in einem Rohrabschnitt angebracht sind, der kürzer ist das Dreifache des Innendurchmessers des Rohrelements. Überraschenderweise kann mit dieser Anordnung eine Zersetzung des PVC vermieden werden und der zusätzliche Druckverlust gering gehalten werden.

Ein Inline-Viskositätsmessverfahren nach einem der vorhergehenden Ausführungsbeispiele kann beispielsweise in einem Verarbeitungsprozess für PVC verwendet werden. Ein derartiger Verarbeitungsprozess für PVC kann einen Extruder umfassen, wobei das PVC mittels des Extruders plastifiziert wird.

Gemäss eines Ausführungsbeispiels enthält der Extruder eine gegenläufig rotierende Doppelschnecke. Insbesondere kann die Doppelschnecke ein erstes und ein zweites Schneckenelement umfassen. Das erste Schneckenelement kann parallel zum zweiten Schneckenelement angeordnet sein. Die ersten und zweiten Schneckenelemente können konisch zueinander angeordnet sein. Gemäss eines Ausführungsbeispiels weist die Doppelschnecke ein erstes Doppelschneckenende und ein zweites Doppelschneckenende auf, wobei das fliessfähige Medium aus dem Extruder am zweiten Doppelschneckenende austritt.

Gemäss eines Ausführungsbeispiels ist zwischen dem zweiten Doppelschneckenende und dem Eintrittsende des Rohrelements ein Übergangsstück angeordnet. Mittels des Übergangsstücks kann der Kanalquerschnitt verändert werden. Beispielsweise kann die Grösse der Querschnittsfläche verändert werden. Beispielsweise kann das Übergangstück ein Übergangsstückeintrittsende und ein Übergangsstückaustrittsende mit einer entsprechenden Querschnittsfläche aufweisen, wobei sich die Querschnittsfläche am Übergangsstückeintrittsende von der Querschnittsfläche am Übergangsstückaustrittsende unterscheidet. Beispielsweise kann die Querschnittsfläche am Übergangsstückeintrittsende grösser als am Übergangsstückaustrittsende sein. Alternativ kann die Querschnittsfläche am Übergangsstückeintrittsende kleiner als am Übergangsstückaustrittsende sein. Gemäss eines Ausführungsbeispiels wird die Form der Querschnittsfläche verändert, beispielsweise wird eine kreisförmige Querschnittsfläche in eine ovale oder polygonale Querschnittsfläche umgewandelt oder umgekehrt. Somit kann mittels des Übergangsstücks die Kanalgeometrie verändert werden. Zwischen dem zweiten Übergangsstückeintrittsende und dem Eintrittsende des Rohrelements kann ein Abstand von maximal dem Vierfachen des Innendurchmessers des Rohrelements vorliegen.

Der Querschnittsfläche des Rohrelements kann rund sein, beispielsweise kreisförmig oder oval, oder eine beliebige polygonale Form aufweisen, beispielsweise quadratisch, rechteckig, fünfeckig, sechseckig, achteckig. Zudem sollte das die Messstrecke enthaltende Rohrelement möglichst direkt im Anschluss an einen Extruder angebracht sein, da dadurch ideale Eingangsströmungsverhältnisse aus der Austragsschnecke in das Rohrelement bestehen, was sich positiv auf die Vermeidung, oder eine Neutralisation der Wandgleiteigenschaften auswirkt. Die Geometrie der Stegelemente kann eine beliebige Form aufweisen. Insbesondere können die Stegelemente beliebig geformte Querschnittsflächen aufweisen, beispielsweise können die Stegelemente eine runde Querschnittsfläche, beispielsweise eine kreisförmige oder ovale Querschnittsfläche aufweisen. Alternativ können die Stegelemente, eine beliebige polygonale Querschnittsfläche aufweisen, beispielsweise eine quadratische, rechteckige, fünfeckige, sechseckige oder achteckige Querschnittsfläche. Stegelemente mit unterschiedlichen Querschnittsflächen können in beliebiger Kombination eingesetzt werden.

Eine erfindungsgemässe Inline-Viskositätsmessvorrichtung zur Messung einer Druckdifferenz zur Ermittlung einer Viskosität eines fliessfähigen Mediums enthält eine in einem Rohrelement angeordnete Messstrecke, welche zur Durchströmung des fliessfähigen Mediums ausgebildet ist. Die Messstrecke ist als ein Strömungskanal ausgebildet. Das Rohrelement umfasst eine Längsachse, ein Eintrittsende und ein Austrittsende. Die Messstrecke erstreckt sich zwischen dem Eintrittsende und dem Austrittsende, wobei in der Messstrecke zumindest ein Einbauelement angeordnet ist. Am Eintrittsende ist ein Eintrittsdrucksensor zur Messung eines Eintrittsdruckmesswerts angeordnet. Ein Austrittsdruck ist am Austrittsende messbar, sodass ein Austrittsdruckmesswert erhältlich ist. Am Austrittsende kann ein Austrittsdrucksensor zur Messung eines Austrittsdruckmesswerts angeordnet sein. Alternativ kann als Austrittsdruckmesswert ein Umgebungsdruck ermittelt werden. Die Inline-Viskositätsmessvorrichtung kann einen Wandler zur Umwandlung des Eintrittsdruckmesswerts und des Austrittsdruckmesswerts in von einer Rechnereinheit verarbeitbare Messgrössen enthalten. Mittels der Rechnereinheit ist aus den Messgrössen ein Differenzdruck zwischen dem Eintrittsdruckmesswert und dem Austrittsdruckmesswert ermittelbar. Das Einbauelement ist als zumindest ein Stegelement ausgebildet, wobei das Stegelement mit einer Stegelementlänge LS in den Strömungskanal hineinragt, die mindestens 25% eines Durchmessers DS des Strömungskanals beträgt.

Gemäss eines Ausführungsbeispiels enthält die Inline-Viskositätsmessvorrichtung einen Durchflusssensor und einen Temperatursensor, wobei mittels des Durchflusssensors ein Durchflussmesswert des durch das Rohrelement strömenden fliessfähigen Mediums ermittelbar ist, wobei mittels des Temperatursensors ein Temperaturmesswert des durch das Rohrelement strömenden fliessfähigen Mediums ermittelbar ist. Anstelle eines Durchflusssensors ist ein mit dem Durchfluss korrelierter Messwert über die Drehzahl eines Schneckenelements eines Extruders ermittelbar. Ein Temperatursensor kann einen Temperaturmesswert des durch das Rohrelement strömenden fliessfähigen Mediums ermitteln. Mittels der Rechnereinheit ist gemäss dieses Ausführungsbeispiels aus dem Differenzdruck, dem Temperaturmesswert und dem Volumenstrommesswert eine Viskosität ermittelbar.

Das Einbauelement ist als zumindest eine Gruppe von Stegelementen ausgebildet, wobei die Gruppe von Stegelementen vorzugsweise eine Mehrzahl von Stegelementen enthält, aber auch ein einziges Stegelement enthalten kann, wenn beispielsweise der Durchmesser des Strömungskanals klein ist.

Die Gruppe von Stegelementen enthält gemäss eines Ausführungsbeispiels mindestens zwei Stegelemente. Gemäss eines Ausführungsbeispiels enthält die Gruppe von Stegelementen mindestens vier Stegelemente. Die Stegelemente der Gruppe von Stegelementen können dadurch gekennzeichnet sein, dass sie parallel zueinander angeordnet sind. Die Stegelemente erstrecken sich insbesondere von einer Innenwand des Rohrelements in den Innenraum des Rohrelements. Die Länge der Stegelemente beträgt insbesondere mindestens ein Viertel, vorzugsweise mindestens ein Drittel des Innendurchmessers des Rohrelements. Der Innendurchmesser des Rohrelements entspricht dem Durchmesser DS des Strömungskanals. Der Innendurchmesser eines nicht kreisförmigen Rohrelements wird als äquivalenter Innendurchmesser gemäss der in der Folge angegebenen Berechnungsvorschrift ermittelt. Die Berechnungsvorschrift gilt sinngemäss auch für den Durchmesser DS des Strömungskanals.

Es hat sich gezeigt, dass mittels der erfindungsgemässen Inline-Viskositätsmessvorrichtung der Druckverlust als Basis für die Bestimmung der Viskosität reproduzierbar und aussagekräftig über derartige, in das fliessfähige Medium eingreifende Stegelemente gemessen werden kann. Dabei hat sich gezeigt, dass Wandgleiten bei den eingreifenden Stegelementen nicht auftritt und sich reproduzierbare Druckverluste messen lassen, die als Basis für die Ermittlung der Viskosität verwendet werden können.

Wenn das fliessfähige Medium Feststoffe enthält, können auch Einflüsse, die durch diese Feststoffe bedingt sind, mit erfasst werden.

Die Stegelemente erstrecken sich in einer ersten Gruppenebene und einer zweiten Gruppenebene, wobei die erste Gruppenebene einen ersten Winkel zur Längsachse des Rohrelements einschliesst und die zweite Gruppenebene einen zweiten Winkel zur Längsachse des Rohrelements einschliesst. Diese Anordnung hat sich als besonders vorteilhaft bezüglich der Messstabilität herausgestellt, insbesondere wenn mindestens ein Teil der Stegelemente derart mit dem Rohrelement verbunden ist, dass zumindest ein Teil der Stegelementenden nicht mit dem Rohrelement verbunden ist.

Gemäss eines Ausführungsbeispiels enthalten die erste Gruppenebene und die zweite Gruppenebene mindestens je ein Stegelement. Gemäss eines Ausführungsbeispiels kann jede der Gruppenebenen mindestens zwei Stegelemente enthalten. Die Stegelemente jeder der Gruppenebenen können dadurch gekennzeichnet sein, dass sie parallel zueinander angeordnet sind.

Gemäss eines Ausführungsbeispiels kreuzt sich die erste Gruppenebene mit der zweiten Gruppenebene.

Gemäss eines Ausführungsbeispiels weist zumindest einer der ersten Winkel und der zweiten Winkel gemessen in Bezug auf die Längsachse einen Wert von ungleich 90 Grad auf. Damit die störenden Wandgleiteigenschaften möglichst wenig in Erscheinung treten, werden somit Stegelemente verwendet, die in einem Winkel ungleich 90 Grad zur Strömungsrichtung angebracht sind. Die Strömungsrichtung entspricht der Längsachse des Rohrelements.

Gemäss eines Ausführungsbeispiels enthalten die erste Gruppenebene und die zweite Gruppenebene mindestens je ein Stegelement.

Gemäss eines Ausführungsbeispiels enthalten zumindest eine der ersten und der zweiten Gruppenebenen zumindest zwei Stegelemente. Insbesondere enthält jede der ersten und zweiten Gruppenebenen mindestens zwei Stegelemente.

Gemäss eines Ausführungsbeispiels sind eine Mehrzahl von Gruppen von Stegelementen in der Messstrecke hintereinander angeordnet.

Gemäss eines Ausführungsbeispiels weist das Rohrelement einen Innendurchmesser auf, wobei zumindest eines der Stegelemente eine Stegelementlänge LS aufweist, die grösser als der Innendurchmesser ist.

Gemäss eines Ausführungsbeispiels beträgt die Stegelementlänge LS maximal das Dreifache des Innendurchmessers. Zudem hat es sich gezeigt, dass es vorteilhaft ist, wenn die Stegelemente in einem Rohrabschnitt angebracht sind, der kürzer ist das Dreifache des Innendurchmessers des Rohrelements. Überraschenderweise kann mit dieser Anordnung eine Zersetzung des PVC vermieden werden und der zusätzliche Druckverlust gering gehalten werden.

Gemäss eines Ausführungsbeispiels ist mindestens ein Teil der Stegelemente derart mit dem Rohrelement verbunden, dass zumindest ein Teil der Stegelementenden nicht mit dem Rohrelement verbunden ist. Das Rohrelement kann mit anderen Worten als Gehäuse für die Stegelemente angesehen werden. Mindestens ein Teil der Stegelemente ist somit derart am Gehäuse befestigt, dass die Stegelementenden zumindest teilweise nicht mit dem Gehäuse verbunden sind. Beispielsweise kann das Stegelement über einen Stegelementarm mit dem Rohrelement verbunden sein. Wenn mehrere Stegelemente vorgesehen sind, können die Stegelemente über einen gemeinsamen Stegelementarm mit dem Rohrelement verbunden sein. Alternativ kann für jedes der Stegelemente ein eigener Stegelementarm vorgesehen sein, mittels welchem es mit dem Rohrelement verbunden ist. Es ist auch möglich, dass die Stegelemente einer ersten Gruppe von Stegelementen und die Stegelemente einer zweiten Gruppe von Stegelementen zumindest einen gemeinsamen Stegelementarm mit dem Rohrelement verbunden sind. Insbesondere kann der gemeinsame Stegelementarm im Kreuzungsbereich verlaufen, wenn sich eine erste Gruppenebene mit Stegelementen mit einer zweiten Gruppenebene mit Stegelementen kreuzt. Eine besonders gute Messstabilität ergibt sich, wenn zumindest ein Teil der Stegelementenden nicht mit dem Rohrelement verbunden sind.

Eine Inline-Viskositätsmessvorrichtung nach einem der vorhergehenden Ausführungsbeispiele kann beispielsweise in einem Verarbeitungsprozess für PVC verwendet werden. Ein derartiger Verarbeitungsprozess für PVC kann einen Extruder umfassen, wobei das PVC mittels des Extruders plastifiziert wird.

Gemäss eines Ausführungsbeispiels enthält der Extruder eine Doppelschnecke. Insbesondere kann die Doppelschnecke ein erstes und ein zweites Schneckenelement umfassen, wobei das erste Schneckenelement parallel zum zweiten Schneckenelement angeordnet ist. Alternativ können das erste Schneckenelement und das zweite Schneckenelement konisch zueinander angeordnet sein.

Gemäss eines Ausführungsbeispiels weist die Doppelschnecke ein erstes Doppelschneckenende und ein zweites Doppelschneckenende auf, wobei das fliessfähige Medium aus dem Extruder am zweiten Doppelschneckenende austritt, wobei zwischen dem zweiten Doppelschneckenende und dem Eintrittsende des Rohrelements ein Abstand von maximal dem Vierfachen des Innendurchmessers des Rohrelements vorliegt.

Die Stegelemente erstrecken sich insbesondere von der Innenwand des Rohrelements in einen Zentrumsbereich des Rohrelements. Unter Zentrumsbereich wird insbesondere ein Bereich verstanden, der die Mittenachse des Rohrelements enthält. Der Zentrumsbereich umfasst insbesondere einen Bereich, welcher als ein zylinderförmiger Bereich mit einem Zentrumsbereichsdurchmesser ausgebildet ist. Der Zentrumsbereichsdurchmesser beträgt maximal die Hälfte des Innendurchmessers des Rohrelements, wobei die Mittenachse des Zentrumsbereichs mit der Mittenachse des Rohrelements zusammenfällt.

Die Stegelemente sind insbesondere derart angeordnet, dass eine Aufteilung des das Rohrelement strömenden fliessfähigen Mediums in mehrere Teilströme erfolgen kann, wobei durch die Teilströme die gesamte freie Querschnittsfläche des Rohrelements nutzbar ist. Mit freier Querschnittsfläche ist der Anteil an der Querschnittsfläche gemeint, welcher für das fliessfähige Medium zur Verfügung steht. Die freie Querschnittsfläche enthält keine Stegelemente und ergibt sich aus der Differenz der Querschnittsfläche des Rohrelements abzüglich der Querschnittsfläche der Stegelemente. Erfindungsgemäss wird das Rohrelement somit insbesondere im Zentrumsbereich vom fliessfähigen Medium durchströmt.

Mittels eines erfindungsgemässen Rohrelements ist es somit möglich, dass Schichten im fliessfähigen Medium ausgebildet werden können. Diese Schichten können mittels der Stegelemente umgelagert werden. Das Umlagern der Schichten führt zu einer Vermeidung der Wandgleiteigenschaften.

Gemäss eines Ausführungsbeispiels enthält das Rohrelement zumindest ein Stegelement. Gemäss eines Ausführungsbeispiels verläuft das zumindest eine Stegelement im Innenraum des Rohrelements. Insbesondere kann eine Gruppe als eine Mehrzahl von Stegelementen ausgebildet sein, wobei die Mehrzahl von Stegelementen im Innenraum des Rohrelements verlaufen. Gemäss eines Ausführungsbeispiels ist zumindest ein Teil der Stegelemente kreuzweise zu einem anderen Teil der Stegelemente angeordnet.

Gemäss eines Ausführungsbeispiels sind zumindest ein Teil der Stegelemente mit einem Gehäuse verbunden, durch welches das Rohrelement ausgebildet wird.

Die Querschnittsfläche des Rohrelements kann rund sein, beispielsweise kreisförmig oder oval, oder eine beliebige polygonale Form aufweisen, beispielsweise quadratisch, rechteckig, fünfeckig, sechseckig, achteckig. Wenn die Querschnittsfläche eine polygonale Form aufweist, kann der mittlere Durchmesser derselben aus der Querschnittsfläche über die Formel für die Fläche eines Kreises bestimmt werden.

Das Einbauelement oder jedes der Einbauelemente kann insbesondere eine erste Gruppe von Stegelementen sowie eine zweite Gruppe von Stegelementen umfassen, wobei die erste Gruppe von Stegelementen sich entlang einer gemeinsamen ersten Gruppenebene erstreckt und die zweite Gruppe von Stegelementen sich entlang einer zweiten gemeinsamen Gruppenebene erstreckt. Die Gruppenebene ist dadurch charakterisiert, dass sie die Mittenachse der Stegelemente enthält. Zumindest ein Teil der Stegelemente erstreckt sich somit den gesamten Innendurchmesser des Rohrelements.

Der Innendurchmesser entspricht einem mittleren Durchmesser, wenn das Rohrelement als ein Kreisrohr ausgeführt ist. Der mittlere Durchmesser für ein eckiges Rohrelement wird als dessen Umfang / n definiert, es handelt sich somit um einen äquivalenten Durchmesser.

Ein Stegelement ist in seinen Abmessungen durch seine Länge, seine Breite und seine Dicke bestimmt. Die Länge des Stegelements wird vom ersten Ende des Stegelements zum zweiten Ende des Stegelements gemessen.

Die Breite des Stegelements wird im Wesentlichen quer zur Strömungsrichtung gemessen. Das heisst, die Breite erstreckt sich im Wesentlichen in einer Ebene, die normal, also in einem Winkel von 90 Grad, zur Länge des Stegelements verläuft und den Querschnitt des Stegelements zeigt. Der Querschnitt des Stegelements wird durch dessen Breite und dessen Dicke charakterisiert. Die Länge zumindest des längsten Stegelements ist mindestens 5-mal so gross wie dessen Breite.

Die Breite des Stegelements ist vorteilhafterweise 0.2-mal bis 8-mal so gross wie dessen Dicke. Wenn die Breite des Stegelements 0.5-mal bis 4-mal so gross wie dessen Dicke ist, ergibt sich ein besonders bevorzugter Bereich, in welchem der Einfluss der Wandgleiteigenschaften ein Minimum aufweist. Die Breite des Stegelements wird als Normalabstand definiert, welcher sich von der ersten Kante und der zweiten Kante des Stegelements auf der Anströmseite erstreckt. Die Breite des Stegelements auf der Anströmseite kann sich von der Breite gemessen auf der Abströmseite des Stegelements unterscheiden.

Unter Kante wird die vom Fluid angeströmte und umströmte Kante des Stegelements verstanden, welche sich im Wesentlichen parallel zur Länge des Stegelements erstreckt. Die Dicke des Stegelements kann variabel sein. Dabei liegt die minimale Dicke, um weniger als 75% und vorteilhafterweise um weniger als 50% unter der maximalen Dicke. Die Variationen können beispielsweise durch Rippen, durch Einbuchtungen, durch Noppen, durch keilförmige Stege oder eine andere Unebenheit bedingt sein.

Das Stegelement ist dadurch charakterisiert, dass in der Strömungsrichtung ebene Flächen oder konkave Flächen vorliegen, die eine Angriffsfläche für das strömende fliessfähige Medium bieten. Diese in Strömungsrichtung ausgerichteten Flächen bewirken, dass gegenüber einem Stegelement, das als ein Rohrelement mit kreisförmiger Querschnittsfläche ausgebildet ist, ein erhöhter Abströmwiderstand entstehen kann.

Der Übergang von zumindest einem der ersten und zweiten Enden des Stegelements zur Innenwand des Rohrelements kann insbesondere fliessend ausgebildet sein. Die Stegelemente und das Rohrelement können demnach aus einem einzigen Bauteil bestehen, welches vorzugsweise durch ein Giessverfahren hergestellt ist. Insbesondere können im Übergangsbereich vom Stegelement zum Rohrelement an den Kanten Rundungen vorgesehen sein, sodass der Fluss des giessfähigen Materials während des Herstellungsverfahrens des Rohrelements für die Vorrichtung nicht beeinträchtigt wird.

### Kurzbeschreibung der Zeichnungen

Nachfolgend wird die erfindungsgemässe Inline-Viskositätsmessvorrichtung anhand einiger Ausführungsbeispiele dargestellt. Es zeigen
Fig. 1a eine Ansicht einer Inline-Viskositätsmessvorrichtung nach einem ersten Ausführungsbeispiel, das nicht unter den Schutzumfang der Ansprüche fällt,
Fig. 1b eine Schnittansicht des Rohrelements gemäss Fig. 1a,
Fig. 2a eine Ansicht einer Inline-Viskositätsmessvorrichtung nach einem zweiten Ausführungsbeispiel,
Fig. 2b eine Schnittansicht des Rohrelements gemäss Fig. 2a,
Fig. 3a eine Ansicht einer Inline-Viskositätsmessvorrichtung nach einem dritten Ausführungsbeispiel,
Fig. 3b eine Schnittansicht des Rohrelements gemäss Fig. 3a,
Fig. 4a eine Ansicht einer Inline-Viskositätsmessvorrichtung nach einem vierten Ausführungsbeispiel,
Fig. 4b eine Schnittansicht des Rohrelements gemäss Fig. 4a,
Fig. 5a eine Ansicht einer Inline-Viskositätsmessvorrichtung nach einem fünften Ausführungsbeispiel, das nicht unter den Schutzumfang der Ansprüche fällt,
Fig. 5b eine Schnittansicht des Rohrelements gemäss Fig. 5a.
Fig. 6a eine Ansicht einer Inline-Viskositätsmessvorrichtung nach einem sechsten Ausführungsbeispiel,
Fig. 6b eine Schnittansicht des Rohrelements gemäss Fig. 6a.

### Detaillierte Beschreibung der Zeichnungen

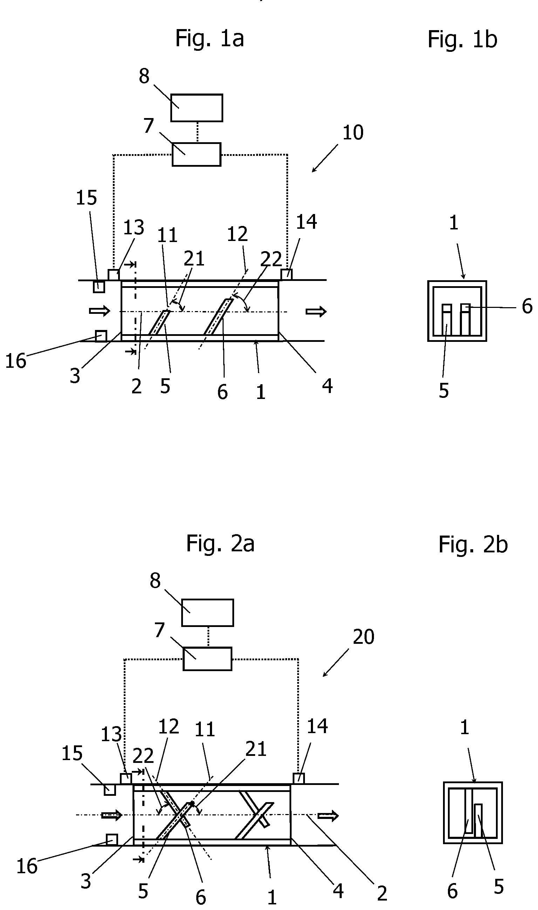

Fig. 1a zeigt eine Inline-Viskositätsmessvorrichtung 10 gemäss eines ersten Ausführungsbeispiels, das nicht unter den Schutzumfang der Ansprüche fällt. Die Inline-Viskositätsmessvorrichtung 10 zur Messung einer Druckdifferenz zur Ermittlung einer Viskosität eines fliessfähigen Mediums enthält eine in einem Rohrelement 1 angeordnete Messstrecke, welche zur Durchströmung des fliessfähigen Mediums ausgebildet ist. Die Messstrecke ist als ein Strömungskanal ausgebildet. Das Rohrelement 1 umfasst eine Längsachse 2, ein Eintrittsende 3 und ein Austrittsende 4. Die Messstrecke erstreckt sich zwischen dem Eintrittsende 3 und dem Austrittsende 4, wobei in der Messstrecke zumindest ein Einbauelement 5,6 angeordnet ist. Am Eintrittsende 3 ist ein Eintrittsdrucksensor 13 zur Messung eines Eintrittsdruckmesswerts angeordnet. Am Austrittsende 4 ist ein Austrittsdrucksensor 14 zur Messung eines Austrittsdruckmesswerts angeordnet. Alternativ kann am Austrittsende ein Umgebungsdruck ermittelt werden. Die Vorrichtung 10 enthält einen Wandler 7 zur Umwandlung des Eintrittsdruckmesswerts und des Austrittsdruckmesswerts in von einer Rechnereinheit 8 verarbeitbare Messgrössen, sodass mittels der Rechnereinheit 8 aus den Messgrössen ein Differenzdruck zwischen dem Eintrittsdruckmesswert und dem Austrittsdruckmesswert ermittelbar ist. Die Inline-Viskositätsmessvorrichtung 10 enthält gemäss des vorliegenden Ausführungsbeispiels zusätzlich einen optionalen Durchflusssensor 15 und einen optionalen Temperatursensor 16, wobei mittels des Durchflusssensors 15 ein Durchflussmesswert des durch das Rohrelement 1 strömenden fliessfähigen Mediums ermittelbar ist. Mittels des Temperatursensors 16 ist ein Temperaturmesswert des durch das Rohrelement 1 strömenden fliessfähigen Mediums ermittelbar, wobei mittels der Rechnereinheit 8 aus dem Differenzdruck, dem Temperaturmesswert und dem Durchflussmesswert eine Viskosität ermittelbar ist. Gemäss des vorliegenden Ausführungsbeispiels sind ein erstes Einbauelement 5 und ein zweites Einbauelement 6 vorgesehen. Insbesondere ist jedes der Einbauelemente 5, 6 als zumindest eine Gruppe von Stegelementen ausgebildet.

Die Stegelemente des Einbauelements 5 erstrecken sich in einer ersten Gruppenebene 11. Die Stegelemente des Einbauelements 6 erstrecken sich einer zweiten Gruppenebene 12. Die erste Gruppenebene 11 schliesst einen ersten Winkel 21 mit der Längsachse des Rohrelements ein. Die zweite Gruppenebene 12 schliesst einen zweiten Winkel 22 mit der Längsachse 2 des Rohrelements 1 ein. Der erste Winkel 21 kann mit dem zweiten Winkel 22 übereinstimmen. Gemäss eines nicht dargestellten Ausführungsbeispiels kann sich der erste Winkel 21 vom zweiten Winkel 22 unterscheiden.

Die Stegelemente des ersten Einbauelements 5 und des zweiten Einbauelements 6 erstrecken sich von der Innenwand des Rohrelements 1 in den Innenraum des Rohrelements 1. Gemäss des vorliegenden Ausführungsbeispiels weisen die Stegelemente des ersten Einbauelements 5 eine sich von den Stegelementen des zweiten Einbauelements 6 unterscheidende Länge auf. Gemäss des vorliegenden, exemplarischen Ausführungsbeispiels sind die Stegelemente des zweiten Einbauelements 6 zumindest teilweise länger als die Stegelemente des ersten Einbauelements 5. In der Darstellung sind die beiden Stegelemente des zweiten Einbauelements 6 länger als die beiden Stegelemente des ersten Einbauelements 5. Gemäss eines nicht dargestellten Ausführungsbeispiels sind die Stegelemente des zweiten Einbauelements 6 zumindest teilweise kürzer als die Stegelemente des ersten Einbauelements 5.

Selbstverständlich kann zumindest eines der ersten oder zweiten Einbauelemente 5, 6 mehr als zwei Stegelemente enthalten, beispielsweise drei, vier, fünf, sechs, sieben, acht Stegelemente.

Selbstverständlich kann auch nur ein einziges Einbauelement vorgesehen sein, entweder ein erstes Einbauelement 5 oder ein zweites Einbauelement 6 oder ein Einbauelement, welches von der Innenwand bis zur gegenüberliegenden Innenwand erstreckt, was in Fig. 1a nicht zeichnerisch dargestellt ist.

Fig. 1b zeigt eine Schnittansicht des Rohrelements 1 gemäss Fig. 1a, der im Bereich des Eintrittsendes 3 gelegt worden ist, wobei die Schnittebene mit einer strichpunktierten Linie und Pfeilen dargestellt ist. In der Schnittansicht ist das erste Einbauelement 5 sichtbar. Das erste Einbauelement 5 ist aus zwei Stegelementen ausgebildet. Die Stegelemente des ersten Einbauelements 5 haben Mittenachsen, die in der ersten Gruppenebene 11 liegen. Das zweite Einbauelement 6 ist dahinter liegend angeordnet, daher in dieser Schnittansicht teilweise durch das erste Einbauelement 5 verdeckt. Die Stegelemente des zweiten Einbauelements 6 haben Mittenachsen, die in der zweiten Gruppenebene 12 liegen.

Gemäss des in Fig. 1a und Fig. 1b dargestellten ersten Ausführungsbeispiels verläuft die erste Gruppenebene 11 im Wesentlichen parallel zur zweiten Gruppenebene 12. Mit anderen Worten sind die ersten Winkel 21 und die zweiten Winkel 22 gleich, wenn das Rohrelement 1 krümmungsfrei ausgebildet ist. Mit anderen Worten bildet die Längsachse 2 des Rohrelements 1 eine Gerade aus.

Fig. 2a zeigt eine Inline-Viskositätsmessvorrichtung 20 gemäss eines zweiten Ausführungsbeispiels der Erfindung. Für gleichwirkende Bauelemente werden dieselben Bezugszeichen wie im ersten Ausführungsbeispiel verwendet. Die Inline-Viskositätsmessvorrichtung 20 zur Messung einer Druckdifferenz zur Ermittlung einer Viskosität eines fliessfähigen Mediums enthält eine in einem Rohrelement 1 angeordnete Messstrecke, welche zur Durchströmung des fliessfähigen Mediums ausgebildet ist. Die Messstrecke ist als ein Strömungskanal ausgebildet. Das Rohrelement 1 umfasst eine Längsachse 2, ein Eintrittsende 3 und ein Austrittsende 4. Die Messstrecke erstreckt sich zwischen dem Eintrittsende 3 und dem Austrittsende 4, wobei in der Messstrecke zumindest ein Einbauelement 5,6 angeordnet ist. Gemäss des vorliegenden Ausführungsbeispiels sind zwei Anordnungen von Einbauelementen 5, 6 hintereinander angeordnet.

Am Eintrittsende 3 ist ein Eintrittsdrucksensor 13 zur Messung eines Eintrittsdruckmesswerts angeordnet. Am Austrittsende 4 ist ein Austrittsdrucksensor 14 zur Messung eines Austrittsdruckmesswerts angeordnet. Alternativ kann am Austrittsende ein Umgebungsdruck ermittelt werden. Die Inline-Viskositätsmessvorrichtung 20 enthält einen Wandler 7 zur Umwandlung des Eintrittsdruckmesswerts und des Austrittsdruckmesswerts in von einer Rechnereinheit 8 verarbeitbare Messgrössen, sodass mittels der Rechnereinheit 8 aus den Messgrössen ein Differenzdruck zwischen dem Eintrittsdruckmesswert und dem Austrittsdruckmesswert ermittelbar ist. Die Vorrichtung 20 enthält gemäss des vorliegenden Ausführungsbeispiels einen optionalen Durchflusssensor 15 und einen optionalen Temperatursensor 16, wobei mittels des Durchflusssensors 15 ein Volumenstrommesswert des durch das Rohrelement 1 strömenden fliessfähigen Mediums ermittelbar ist. Mittels des Temperatursensors 16 ist ein Temperaturmesswert des durch das Rohrelement 1 strömenden fliessfähigen Mediums ermittelbar, wobei mittels der Rechnereinheit 8 aus dem Differenzdruck, dem Temperaturmesswert und dem Durchflussmesswert eine Viskosität ermittelbar ist.

Jedes der Einbauelemente 5, 6 ist gemäss dieses Ausführungsbeispiels als zumindest eine Gruppe von Stegelementen ausgebildet. Die Stegelemente, welche das erste Einbauelement 5 ausbilden, erstrecken sich in einer ersten Gruppenebene 11. Die Stegelemente, welche das zweite Einbauelement 6 ausbilden, erstrecken sich einer zweiten Gruppenebene 12. Die erste Gruppenebene 11 schliesst einen ersten Winkel 21 mit der Längsachse des Rohrelements 1 ein. Die zweite Gruppenebene 12 schliesst einen zweiten Winkel 22 mit der Längsachse 2 des Rohrelements 1 ein. Gemäss des vorliegenden Ausführungsbeispiels kreuzt sich die erste Gruppenebene 11 mit der zweiten Gruppenebene 12.

Das erste Einbauelement 5 besteht gemäss dieses Ausführungsbeispiels aus einem einzigen Stegelement. Das Stegelement des ersten Einbauelements 5 hat eine Mittenachse, die in der ersten Gruppenebene 11 liegt. Das zweite Einbauelement 6 ist dahinter liegend angeordnet, daher in dieser Schnittansicht teilweise durch das erste Einbauelement 5 verdeckt. Das Stegelement des zweiten Einbauelements 6 hat eine Mittenachse, die in der zweiten Gruppenebene 12 liegt. Die erste Gruppenebene 11 kreuzt sich mit der zweiten Gruppenebene 12. Mit anderen Worten kreuzen sich die ersten und zweiten Einbauelemente 5, 6. Die ersten und zweiten Einbauelemente 5, 6 haben Stegelemente, die nur mit einem einzigen Ende mit der Rohrinnenwand verbunden sind. Das gegenüberliegende Ende der Stegelemente ist insbesondere in einem Abstand zur gegenüberliegenden Innenwand angeordnet, der grösser ist als der Wandabstand, in welchem ein Wandgleiten auftritt. Das gegenüberliegende Ende wird nachfolgend als das freie Ende bezeichnet. Insbesondere kann der Abstand des freien Endes zumindest eines der Stegelemente mindestens ein Zehntel des Innendurchmessers des Rohrelements betragen.

Das Stegelement des ersten Einbauelements 5 kann sich in seiner Länge vom Stegelement des zweiten Einbauelements 6 unterscheiden.

In Fig. 2a ist eine erste Anordnung und eine zweite Anordnung dargestellt. Die erste Anordnung besteht aus den Einbauelementen 5, 6. Die zweite Anordnung besteht gemäss dieses Ausführungsbeispiels ebenfalls aus gleichartigen Einbauelementen 5, 6, die in Fig. 2a nicht bezeichnet sind und in Fig. 2b nicht sichtbar sind, da sie von den Einbauelementen 5, 6 der stromaufwärts liegenden Anordnung verdeckt sind.

Fig. 2b zeigt eine Schnittansicht des Rohrelements 1 gemäss Fig. 2a, der im Bereich des Eintrittsendes 3 gelegt worden ist, wobei die Schnittebene mit einer strichpunktierten Linie und Pfeilen dargestellt ist. In der Schnittansicht sind das erste Einbauelement 5 und das zweite Einbauelement 6 der ersten Anordnung sichtbar.

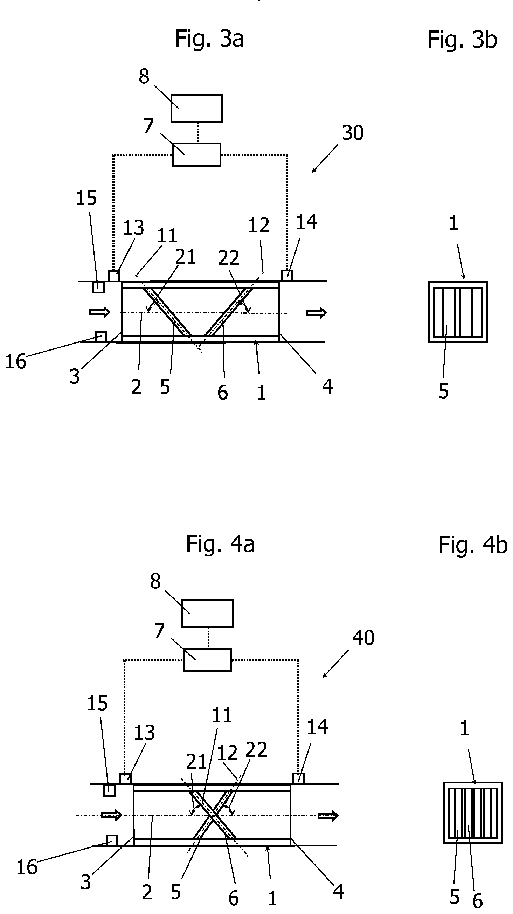

Fig. 3a zeigt eine Inline-Viskositätsmessvorrichtung 30 gemäss eines dritten Ausführungsbeispiels der Erfindung. Die Inline-Viskositätsmessvorrichtung 30 zur Messung einer Druckdifferenz zur Ermittlung einer Viskosität eines fliessfähigen Mediums enthält eine in einem Rohrelement 1 angeordnete Messstrecke, welche zur Durchströmung des fliessfähigen Mediums ausgebildet ist. Die Messstrecke ist als ein Strömungskanal ausgebildet. Das Rohrelement 1 umfasst eine Längsachse 2, ein Eintrittsende 3 und ein Austrittsende 4. Die Messstrecke erstreckt sich zwischen dem Eintrittsende 3 und dem Austrittsende 4, wobei in der Messstrecke zumindest ein Einbauelement 5, 6 angeordnet ist. Am Eintrittsende 3 ist ein Eintrittsdrucksensor 13 zur Messung eines Eintrittsdruckmesswerts angeordnet. Am Austrittsende 4 ist ein Austrittsdrucksensor 14 zur Messung eines Austrittsdruckmesswerts angeordnet. Alternativ kann am Austrittsende ein Umgebungsdruck ermittelt werden. Die Inline-Viskositätsmessvorrichtung 30 enthält einen Wandler 7 zur Umwandlung des Eintrittsdruckmesswerts und des Austrittsdruckmesswerts in von einer Rechnereinheit 8 verarbeitbare Messgrössen, sodass mittels der Rechnereinheit 8 aus den Messgrössen ein Differenzdruck zwischen dem Eintrittsdruckmesswert und dem Austrittsdruckmesswert ermittelbar ist. Die Vorrichtung 30 enthält gemäss des vorliegenden Ausführungsbeispiels zusätzlich einen optionalen Durchflusssensor 15 und einen optionalen Temperatursensor 16, wobei mittels des Durchflusssensors 15 ein Durchflussmesswert des durch das Rohrelement 1 strömenden fliessfähigen Mediums ermittelbar ist. Mittels des Temperatursensors 16 ist ein Temperaturmesswert des durch das Rohrelement 1 strömenden fliessfähigen Mediums ermittelbar, wobei mittels der Rechnereinheit 8 aus dem Differenzdruck, dem Temperaturmesswert und dem Durchflussmesswert eine Viskosität ermittelbar ist.

Gemäss des vorliegenden Ausführungsbeispiels sind ein erstes Einbauelement 5 und ein zweites Einbauelement 6 vorgesehen. Jedes der Einbauelemente 5, 6 kann als zumindest eine Gruppe von Stegelementen ausgebildet sein, die sich in einer ersten Gruppenebene 11 und einer zweiten Gruppenebene 12 erstrecken. Die erste Gruppenebene 11 schliesst einen ersten Winkel 21 mit der Längsachse des Rohrelements ein und die zweite Gruppenebene 12 schliesst einen zweiten Winkel 22 mit der Längsachse 2 des Rohrelements 1 ein.

Fig. 3b zeigt eine Schnittansicht des Rohrelements 1 gemäss Fig. 3a, der im Bereich des Eintrittsendes 3 gelegt worden ist. In der Schnittansicht ist das erste Einbauelement 5 sichtbar. Das zweite Einbauelement 6 ist dahinter liegend angeordnet, daher in dieser Schnittansicht durch das erste Einbauelement 5 verdeckt. Das erste Einbauelement 5 besteht gemäss des vorliegenden Ausführungsbeispiels aus zwei Stegelementen.

Fig. 4a zeigt eine Inline-Viskositätsmessvorrichtung 40 gemäss eines vierten Ausführungsbeispiels der Erfindung. Für gleichwirkende Bauelemente werden dieselben Bezugszeichen wie im ersten Ausführungsbeispiel verwendet. Die Inline-Viskositätsmessvorrichtung 40 zur Messung einer Druckdifferenz zur Ermittlung einer Viskosität eines fliessfähigen Mediums enthält eine in einem Rohrelement 1 angeordnete Messstrecke, welche zur Durchströmung des fliessfähigen Mediums ausgebildet ist. Die Messstrecke ist als ein Strömungskanal ausgebildet. Das Rohrelement 1 umfasst eine Längsachse 2, ein Eintrittsende 3 und ein Austrittsende 4. Die Messstrecke erstreckt sich zwischen dem Eintrittsende 3 und dem Austrittsende 4, wobei in der Messstrecke zumindest ein Einbauelement 5,6 angeordnet ist. Am Eintrittsende 3 ist ein Eintrittsdrucksensor 13 zur Messung eines Eintrittsdruckmesswerts angeordnet. Am Austrittsende 4 ist ein Austrittsdrucksensor 14 zur Messung eines Austrittsdruckmesswerts angeordnet. Alternativ kann am Austrittsende ein Umgebungsdruck ermittelt werden. Die Vorrichtung 40 enthält einen Wandler 7 zur Umwandlung des Eintrittsdruckmesswerts und des Austrittsdruckmesswerts in von einer Rechnereinheit 8 verarbeitbare Messgrössen, sodass mittels der Rechnereinheit 8 aus den Messgrössen ein Differenzdruck zwischen dem Eintrittsdruckmesswert und dem Austrittsdruckmesswert ermittelbar ist. Die Vorrichtung 40 enthält gemäss des vorliegenden Ausführungsbeispiels zusätzlich einen optionalen Durchflusssensor 15 und einen optionalen Temperatursensor 16, wobei mittels des Durchflusssensors 15 ein Durchflussmesswert des durch das Rohrelement 1 strömenden fliessfähigen Mediums ermittelbar ist. Mittels des Temperatursensors 16 ist ein Temperaturmesswert des durch das Rohrelement 1 strömenden fliessfähigen Mediums ermittelbar, wobei mittels der Rechnereinheit 8 aus dem Differenzdruck, dem Temperaturmesswert und dem Durchflussmesswert eine Viskosität ermittelbar ist.

Das Einbauelement 5, 6 ist als zumindest eine Gruppe von Stegelementen ausgebildet, die sich in einer ersten Gruppenebene 11 und einer zweiten Gruppenebene 12 erstrecken, wobei die erste Gruppenebene 11 einen ersten Winkel 21 mit der Längsachse des Rohrelements einschliesst und die zweite Gruppenebene 12 einen zweiten Winkel 22 mit der Längsachse 2 des Rohrelements 1 einschliesst. Gemäss des vorliegenden Ausführungsbeispiels kreuzt sich die erste Gruppenebene 11 mit der zweiten Gruppenebene 12.

Fig. 4b zeigt eine Schnittansicht des Rohrelements 1 gemäss Fig. 4a, der im Bereich des Eintrittsendes 3 gelegt worden ist. In der Schnittansicht sind das erste Einbauelement 5 und das zweite Einbauelement 6 sichtbar. Gemäss dieses exemplarischen Ausführungsbeispiels bestehen das Einbauelement 5 aus zwei Stegelementen und das Einbauelement 6 aus zwei Stegelementen.

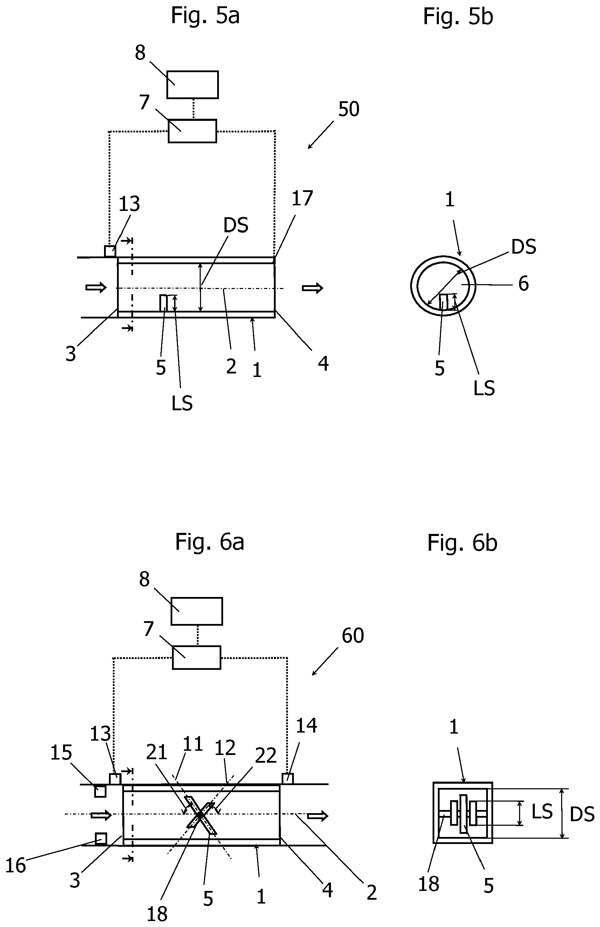

Fig. 5a zeigt eine Inline-Viskositätsmessvorrichtung 50 gemäss eines fünften Ausführungsbeispiels, das nicht unter den Schutzumfang der Ansprüche fällt. Die Inline-Viskositätsmessvorrichtung 50 zur Messung einer Druckdifferenz zur Ermittlung einer Viskosität eines fliessfähigen Mediums enthält eine in einem Rohrelement 1 angeordnete Messstrecke, welche zur Durchströmung des fliessfähigen Mediums ausgebildet ist. Die Messstrecke ist als ein Strömungskanal ausgebildet. Das Rohrelement 1 umfasst eine Längsachse 2, ein Eintrittsende 3 und ein Austrittsende 4. Die Messstrecke erstreckt sich zwischen dem Eintrittsende 3 und dem Austrittsende 4, wobei in der Messstrecke zumindest ein Einbauelement 5 angeordnet ist. Am Eintrittsende 3 ist ein Eintrittsdrucksensor 13 zur Messung eines Eintrittsdruckmesswerts angeordnet. Am Austrittsende 4 wird ein Austrittsdruckmesswert 17 ermittelt. Gemäss des vorliegenden Ausführungsbeispiels wird am Austrittsende ein Umgebungsdruck ermittelt. Die Inline-Viskositätsmessvorrichtung 50 enthält einen Wandler 7 zur Umwandlung des Eintrittsdruckmesswerts und des Austrittsdruckmesswerts in von einer Rechnereinheit 8 verarbeitbare Messgrössen, sodass mittels der Rechnereinheit 8 aus den Messgrössen ein Differenzdruck zwischen dem Eintrittsdruckmesswert und dem Austrittsdruckmesswert ermittelbar ist. Die Inline-Viskositätsmessvorrichtung 50 kann zusätzlich einen optionalen Durchflusssensor und/oder einen optionalen Temperatursensor enthalten. Alternativ kann anstelle eines Durchflusssensors ein anderer Messwertaufnehmer für den Durchflussmesswert vorgesehen werden. Beispielsweise kann eine Drehzahl eines Schneckenelements eines Extruders ermittelt werden. Mittels des Durchflusssensors oder eines anderen Messwertaufnehmers kann ein Durchflussmesswert des durch das Rohrelement 1 strömenden fliessfähigen Mediums ermittelt werden. Mittels des optionalen Temperatursensors ist ein Temperaturmesswert des durch das Rohrelement 1 strömenden fliessfähigen Mediums ermittelbar, wobei mittels der Rechnereinheit 8 aus dem Differenzdruck, gegebenenfalls dem Temperaturmesswert und gegebenenfalls dem Durchflussmesswert eine Viskosität ermittelbar ist.

Gemäss des vorliegenden Ausführungsbeispiels ist nur ein erstes Einbauelement 5 vorgesehen. Insbesondere ist das Einbauelement 5 als zumindest ein Stegelement ausgebildet. Das Stegelement ragt mit einer Stegelementlänge LS in den Strömungskanal hinein, die mindestens 25% eines Durchmessers DS des Strömungskanals beträgt.

Das Stegelement oder die Stegelemente des ersten Einbauelements 5 erstrecken sich von der Innenwand des Rohrelements 1 in den Innenraum des Rohrelements 1 bzw. des Strömungskanals.

Selbstverständlich kann das Einbauelement 5 zwei Stegelemente oder mehr als zwei Stegelemente enthalten, beispielsweise drei, vier, fünf, sechs, sieben, acht Stegelemente.

Selbstverständlich kann sich das Einbauelement von der Innenwand bis zur gegenüberliegenden Innenwand erstrecken, was in Fig. 5a oder Fig. 5b nicht zeichnerisch dargestellt ist.

Fig. 5b zeigt eine Schnittansicht des Rohrelements 1 gemäss Fig. 5a, der im Bereich des Eintrittsendes 3 gelegt worden ist, wobei die Schnittebene mit einer strichpunktierten Linie und Pfeilen dargestellt ist. In der Schnittansicht ist das erste Einbauelement 5 sichtbar. Das erste Einbauelement 5 besteht gemäss dieses exemplarischen Ausführungsbeispiels aus einem einzigen Stegelement.

Das zumindest eine Stegelement des Einbauelements 5 kann einen Winkel von 90 Grad in Bezug auf die Längsachse 2 einschliessen, wie in Fig. 5a gezeigt. Der Winkel kann auch von 90 Grad abweichen, was zeichnerisch nicht dargestellt ist.

Fig. 6a zeigt eine Inline-Viskositätsmessvorrichtung 60 gemäss eines sechsten Ausführungsbeispiels der Erfindung. Die Inline-Viskositätsmessvorrichtung 60 zur Messung einer Druckdifferenz zur Ermittlung einer Viskosität eines fliessfähigen Mediums enthält eine in einem Rohrelement 1 angeordnete Messstrecke, welche zur Durchströmung des fliessfähigen Mediums ausgebildet ist. Die Messstrecke ist als ein Strömungskanal ausgebildet. Das Rohrelement 1 umfasst eine Längsachse 2, ein Eintrittsende 3 und ein Austrittsende 4. Die Messstrecke erstreckt sich zwischen dem Eintrittsende 3 und dem Austrittsende 4, wobei in der Messstrecke zumindest ein Einbauelement 5 angeordnet ist. Am Eintrittsende 3 ist ein Eintrittsdrucksensor 13 zur Messung eines Eintrittsdruckmesswerts angeordnet. Am Austrittsende 4 ist ein Austrittsdrucksensor 14 zur Messung eines Austrittsdruckmesswerts angeordnet. Alternativ kann am Austrittsende ein Umgebungsdruck ermittelt werden, wie in Fig. 5a gezeigt. Die Inline-Viskositätsmessvorrichtung 60 enthält einen Wandler 7 zur Umwandlung des Eintrittsdruckmesswerts und des Austrittsdruckmesswerts in von einer Rechnereinheit 8 verarbeitbare Messgrössen, sodass mittels der Rechnereinheit 8 aus den Messgrössen ein Differenzdruck zwischen dem Eintrittsdruckmesswert und dem Austrittsdruckmesswert ermittelbar ist. Die Inline-Viskositätsmessvorrichtung 60 kann zusätzlich einen optionalen Durchflusssensor 15 und/oder einen optionalen Temperatursensor 16 enthalten. Alternativ kann anstelle eines Durchflusssensors ein anderer Messwertaufnehmer für den Durchflussmesswert vorgesehen werden. Beispielsweise kann eine Drehzahl eines Schneckenelements eines Extruders ermittelt werden. Mittels des Durchflusssensors oder eines anderen Messwertaufnehmers kann ein Durchflussmesswert des durch das Rohrelement 1 strömenden fliessfähigen Mediums ermittelt werden. Mittels des optionalen Temperatursensors ist ein Temperaturmesswert des durch das Rohrelement 1 strömenden fliessfähigen Mediums ermittelbar, wobei mittels der Rechnereinheit 8 aus dem Differenzdruck, gegebenenfalls dem Temperaturmesswert und gegebenenfalls dem Durchflussmesswert eine Viskosität ermittelbar ist.

Gemäss des vorliegenden Ausführungsbeispiels ist nur ein erstes Einbauelement 5 vorgesehen. Insbesondere enthält das Einbauelement 5 zumindest ein Stegelement. Das Stegelement ragt mit einer Stegelementlänge LS in den Strömungskanal hinein, die mindestens 25% eines Durchmessers DS des Strömungskanals beträgt, siehe hierzu auf Fig. 6b.

Das Stegelement oder die Stegelemente des ersten Einbauelements 5 erstrecken sich von der Mittenachse des Rohrelements 1 in den Innenraum des Rohrelements 1 bzw. des Strömungskanals. Gemäss des vorliegenden Ausführungsbeispiels weist das Stegelement bzw. jedes der Stegelemente ein erstes Stegelementende und ein zweites Stegelementende auf, wobei weder das erste Stegelementende noch das zweite Stegelementende mit dem Rohrelement verbunden sind. Insbesondere weisen sowohl das erste Stegelementende als auch das zweite Stegelementende einen Abstand von einer Innenwand des Rohrelements auf, der mindestens 10% des Innendurchmessers des Rohrelements, bzw. des Durchmessers DS des Strömungskanals, entspricht. Nach einem Ausführungsbeispiel enthält das Stegelement einen Stegelementarm 18, welcher als ein Verbindungselement mit der Innenwand des Rohrelements ausgebildet ist. In Fig. 6a ist einer der beiden Stegelementarme 18 im Schnitt dargestellt, da die vordere Seitenwand des Rohrelements 1 vor der Schnittebene liegt und daher in Fig. 6a weggeschnitten ist.

Nach einem Ausführungsbeispiel sind mindestens 60% der Querschnittsfläche des Rohrelements durch das oder die Stegelemente abgedeckt. Insbesondere sind mindestens 60% der Querschnittsfläche und maximal 90% der Querschnittsfläche des Rohrelements durch das oder die Stegelemente abgedeckt. Insbesondere können mindestens zwei Stegelemente vorgesehen sein. Wenn zwei oder mehr Stegelemente vorgesehen sind, können sie miteinander über Stegelementarme 18 verbunden sein, wie in Fig. 6b gezeigt.

Das Einbauelement 5 ist nach dem vorliegenden Ausführungsbeispiel als eine Gruppe von Stegelementen ausgebildet, die sich in einer ersten Gruppenebene 11 und einer zweiten Gruppenebene 12 erstrecken, wobei die erste Gruppenebene 11 einen ersten Winkel 21 mit der Längsachse des Rohrelements einschliesst und die zweite Gruppenebene 12 einen zweiten Winkel 22 mit der Längsachse 2 des Rohrelements 1 einschliesst. Gemäss des vorliegenden Ausführungsbeispiels kreuzt sich die erste Gruppenebene 11 mit der zweiten Gruppenebene 12.Selbstverständlich kann das Einbauelement 5 zwei Stegelemente oder mehr als zwei Stegelemente enthalten, beispielsweise drei, vier, fünf, sechs, sieben, acht Stegelemente. Selbstverständlich kann sich das Einbauelement von der Innenwand bis zur gegenüberliegenden Innenwand erstrecken, was in Fig. 6a oder Fig. 6b nicht zeichnerisch dargestellt ist.

Fig. 6b zeigt eine Schnittansicht des Rohrelements 1 gemäss Fig. 6a, der im Bereich des Eintrittsendes 3 gelegt worden ist, wobei die Schnittebene mit einer strichpunktierten Linie und Pfeilen dargestellt ist. In der Schnittansicht ist das erste Einbauelement 5 sichtbar. Das erste Einbauelement 5 besteht gemäss dieses exemplarischen Ausführungsbeispiels aus drei Stegelementen.

Die Stegelemente des Einbauelements 5 können einen Winkel weniger oder mehr als 90 Grad in Bezug auf die Längsachse 2 einschliessen, wie in Fig. 6a gezeigt. Der Winkel kann auch 90 Grad betragen, was zeichnerisch nicht dargestellt ist.

Das Stegelement oder zumindest ein Teil der Stegelemente kann sich gemäss jedes der Ausführungsbeispiele über den gesamten Innendurchmesser des Rohrelements erstrecken.

Der Innendurchmesser kann einem mittleren Durchmesser entsprechen, wenn der Querschnitt des Rohrelements nicht kreisförmig ist. Der mittlere Durchmesser entspricht dem Innendurchmesser, wenn das Rohrelement mit kreisförmiger Querschnittsfläche ausgeführt ist. Der mittlere Durchmesser für ein eckiges oder ovales Rohrelement wird als dessen Umfang / n definiert, es handelt sich somit um einen äquivalenten Durchmesser.

Ein Stegelement ist in seinen Abmessungen durch seine Länge, seine Breite und seine Dicke bestimmt. Die Länge des Stegelements wird vom ersten Ende des Stegelements zum zweiten Ende des Stegelements gemessen.

Die Breite des Stegelements wird im Wesentlichen quer zur Strömungsrichtung gemessen. Das heisst, die Breite erstreckt sich im Wesentlichen in einer Ebene, die normal zur Länge des Stegelements verläuft und den Querschnitt des Stegelements zeigt. Der Querschnitt des Stegelements wird durch dessen Breite und dessen Dicke charakterisiert. Die Länge zumindest des längsten Stegelements ist mindestens 5-mal so gross wie dessen Breite.

Die Breite des Stegelements ist 0.5- bis 5-mal so gross wie dessen Dicke, vorteilhafterweise 0.5- bis 3-mal so gross wie dessen Dicke. Wenn die Breite des Stegelements 0.5 - bis 2-mal so gross wie dessen Dicke ist, ergibt sich ein besonders bevorzugter Bereich, in welchem der Einfluss der Wandgleiteigenschaften ein Minimum aufweist. Die Breite des Stegelements wird als Normalabstand definiert, welcher sich von der ersten Kante und der zweiten Kante des Stegelements auf der Anströmseite erstreckt. Die Breite des Stegelements auf der Anströmseite kann sich von der Breite des Stegelements, die auf der Abströmseite gemessen wird, unterscheiden.

Unter einer Kante wird die vom Fluid angeströmte und umströmte Kante des Stegelements verstanden, welche sich im Wesentlichen parallel zur Länge des Stegelements erstreckt. Die Dicke des Stegelements kann variabel sein. Dabei liegt die minimale Dicke, um weniger als 75% und vorteilhafterweise um weniger als 50% unter der maximalen Dicke. Die Variationen können beispielsweise durch Rippen, durch Einbuchtungen, durch Noppen, durch keilförmige Stege oder andere Profilvariationen oder Unebenheiten bedingt sein.

Das Stegelement ist dadurch charakterisiert, dass in der Strömungsrichtung ebene Oberflächen, konvexe Oberflächen oder konkave Oberflächen vorliegen, die eine Angriffsfläche für das fliessfähige Medium bieten. Diese in Strömungsrichtung ausgerichteten Flächen bewirken, dass gegenüber einem Stegelement, das als ein Rohrelement mit kreisförmiger Querschnittsfläche ausgebildet ist, ein erhöhter Abströmwiderstand entstehen kann.

Der Übergang von zumindest einem der ersten und zweiten Enden des Stegelements zur Innenwand des Rohrelements kann insbesondere fliessend ausgebildet sein. Die Stegelemente und das Rohrelement können demnach aus einem einzigen Bauteil bestehen, welches vorzugsweise durch ein Giessverfahren hergestellt ist. Insbesondere können im Übergangsbereich vom Stegelement zum Rohrelement an den Kanten Rundungen vorgesehen sein, sodass der Fluss des giessfähigen Materials während des Herstellungsverfahrens des Rohrelements für die Vorrichtung nicht beeinträchtigt wird.

Für eine Fachperson ist offensichtlich, dass viele weitere Varianten zusätzlich zu den beschriebenen Verfahren oder Vorrichtungen möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist. Für die Interpretation der Ansprüche oder der Beschreibung ist die breitest mögliche Lesart der Ansprüche massgeblich. Insbesondere sollen die Begriffe "enthalten" oder "beinhalten" derart interpretiert werden, dass sie sich auf Elemente, Komponenten oder Schritte in einer nicht-ausschliesslichen Bedeutung beziehen, wodurch angedeutet werden soll, dass die Elemente, Komponenten oder Schritte vorhanden sein können oder genutzt werden können, dass sie mit anderen Elementen, Komponenten oder Schritten kombiniert werden können, die nicht explizit erwähnt sind. Wenn die Ansprüche sich auf ein Element oder eine Komponente aus einer Gruppe beziehen, die aus A, B, C bis N Elementen oder Komponenten bestehen kann, soll diese Formulierung derart interpretiert werden, dass nur ein einziges Element dieser Gruppe erforderlich ist, und nicht eine Kombination von A und N, B und N oder irgendeiner anderen Kombination von zwei oder mehr Elementen oder Komponenten dieser Gruppe.

## Patentansprüche

1. Inline-Viskositätsmessverfahren zur Messung einer Druckdifferenz zur Bestimmung einer Viskosität eines fliessfähigen Mediums, wobei das fliessfähige Medium eine in einem Rohrelement (1) angeordnete Messstrecke durchströmt, wobei die Messstrecke als ein Strömungskanal ausgebildet ist, wobei das Rohrelement (1) eine Längsachse (2), ein Eintrittsende (3) und ein Austrittsende (4) umfasst, wobei sich die Messstrecke zumindest zwischen dem Eintrittsende (3) und dem Austrittsende (4) erstreckt, wobei in der Messstrecke zumindest ein Einbauelement (5, 6) angeordnet ist, wobei ein Eintrittsdruck am Eintrittsende (3) von einem Eintrittsdrucksensor (13) gemessen wird, sodass ein Eintrittsdruckmesswert erhalten wird, und wobei ein Austrittsdruck am Austrittsende (4) gemessen wird, sodass ein Austrittsdruckmesswert erhalten wird, wobei der Eintrittsdruckmesswert und der Austrittsdruckmesswert mittels eines Wandlers (7) in von einer Rechnereinheit (8) verarbeitbare Messgrössen umgewandelt werden, wobei die Rechnereinheit (8) einen Differenzdruck zwischen dem Eintrittsdruckmesswert und dem Austrittsdruckmesswert ermittelt, wobei das Einbauelement (5, 6) als zumindest ein Stegelement ausgebildet ist, wobei das Stegelement mit einer Stegelementlänge LS in den Strömungskanal hineinragt, die mindestens 25% eines Durchmessers (DS) des Strömungskanals beträgt, **dadurch gekennzeichnet, dass** sich eine Gruppe von Stegelementen in einer ersten Gruppenebene (11) und einer zweiten Gruppenebene (12) erstrecken, wobei die erste Gruppenebene (11) einen ersten Winkel (21) zur Längsachse (2) des Rohrelements (1) einschliesst und die zweite Gruppenebene (12) einen zweiten Winkel (22) zur Längsachse (2) des Rohrelements (1) einschliesst.

2. Verfahren nach einem der vorhergehenden Ansprüche, wobei die erste Gruppenebene (11) sich mit der zweiten Gruppenebene (12) kreuzt.

3. Verfahren nach einem der Ansprüche 1 oder 2, wobei zumindest einer der ersten Winkel (21) und der zweiten Winkel (22) gemessen in Bezug auf die Längsachse (2) einen Wert von ungleich 90 Grad aufweist.

4. Verfahren nach einem der vorhergehenden Ansprüche, wobei die erste Gruppenebene (11) und die zweite Gruppenebene (12) mindestens je ein Stegelement enthalten.

5. Verfahren nach einem der vorhergehenden Ansprüche, wobei eine Mehrzahl von Gruppen von Stegelementen in der Messstrecke hintereinander angeordnet sind.

6. Verfahren nach einem der vorhergehenden Ansprüche, wobei das Rohrelement (1) einen Innendurchmesser aufweist, wobei zumindest eines der Stegelemente eine Stegelementlänge (LS) aufweist, die grösser als der Innendurchmesser ist.

7. Verfahren nach einem der vorhergehenden Ansprüche, wobei mindestens ein Teil der Stegelemente derart mit dem Rohrelement verbunden ist, dass zumindest ein Teil der Stegelementenden nicht mit dem Rohrelement verbunden ist.

8. Verwendung des Inline-Viskositätsmessverfahrens nach einem der vorhergehenden Ansprüche in einem Verarbeitungsprozess für PVC.

9. Verwendung nach Anspruch 8, wobei der Verarbeitungsprozess für PVC einen Extruder umfasst, wobei das PVC mittels des Extruders plastifiziert wird.

10. Verwendung nach Anspruch 9, wobei der Extruder eine gegenläufig rotierende Doppelschnecke enthält.

11. Verwendung nach Anspruch 10, wobei die Doppelschnecke ein erstes Doppelschneckenende und ein zweites Doppelschneckenende aufweist, wobei das fliessfähige Medium aus dem Extruder am zweiten Doppelschneckenende austritt.

12. Verwendung nach Anspruch 11, wobei zwischen dem zweiten Doppelschneckenende und dem Eintrittsende des Rohrelements ein Übergangsstück angeordnet ist, wobei das Übergangsstück ein Übergangsstückeintrittsende und ein Übergangsstückaustrittsende mit einer entsprechenden Querschnittsfläche aufweist, wobei sich die Querschnittsfläche am Übergangsstückeintrittsende sich von der Querschnittsfläche am Übergangsstückaustrittsende unterscheidet.

13. Inline-Viskositätsmessvorrichtung (10) zur Messung einer Druckdifferenz zur Ermittlung einer Viskosität eines fliessfähigen Mediums, enthaltend eine in einem Rohrelement (1) angeordnete Messstrecke, welche zur Durchströmung des fliessfähigen Mediums ausgebildet ist, wobei die Messstrecke als ein Strömungskanal ausgebildet ist, wobei das Rohrelement (1) eine Längsachse (2), ein Eintrittsende (3) und ein Austrittsende (4) umfasst, wobei sich die Messstrecke zumindest zwischen dem Eintrittsende (3) und dem Austrittsende (4) erstreckt, wobei in der Messstrecke zumindest ein Einbauelement (5, 6) angeordnet ist, wobei am Eintrittsende (3) ein Eintrittsdrucksensor (13) zur Messung eines Eintrittsdruckmesswerts angeordnet ist, wobei ein Austrittsdruck am Austrittsende (4) messbar ist, sodass ein Austrittsdruckmesswert erhältlich ist, wobei die Vorrichtung (10) einen Wandler (7) zur Umwandlung des Eintrittsdruckmesswerts und des Austrittsdruckmesswerts in von einer Rechnereinheit (8) verarbeitbare Messgrössen enthält, sodass mittels der Rechnereinheit (8) aus den Messgrössen ein Differenzdruck zwischen dem Eintrittsdruckmesswert und dem Austrittsdruckmesswert ermittelbar ist, wobei das Einbauelement als zumindest ein Stegelement ausgebildet ist, wobei das Stegelement mit einer Stegelementlänge (LS) in den Strömungskanal hineinragt, die mindestens 25% eines Durchmessers (DS) des Strömungskanals beträgt, wobei das Einbauelement als zumindest eine Gruppe von Stegelementen ausgebildet ist, wobei sich die Stegelemente in einer ersten Gruppenebene (11) und einer zweiten Gruppenebene (12) erstrecken, wobei die erste Gruppenebene (11) einen ersten Winkel (21) mit der Längsachse des Rohrelements einschliesst und die zweite Gruppenebene (12) einen zweiten Winkel (22) mit der Längsachse (2) des Rohrelements (1) einschliesst.

## Claims

1. Inline viscosity measuring method for measuring a pressure difference for determining a viscosity of a flowable medium, wherein the flowable medium flows through a measuring section arranged in a tubular element (1), wherein the measuring section is configured as a flow channel, wherein the tubular element (1) comprises a longitudinal axis (2), an inlet end (3) and an outlet end (4), wherein the measuring section extends at least between the inlet end (3) and the outlet end (4), wherein at least one installation element (5, 6) is arranged in the measuring section, wherein an inlet pressure at the inlet end (3) is measured by an inlet pressure sensor (13), so that a measured inlet pressure value is obtained, and wherein an outlet pressure is measured at the outlet end (4) so that a measured outlet pressure value is obtained, wherein the measured inlet pressure value and the measured outlet pressure value are converted by means of a transducer (7) into measured variables which can be processed by a computer unit (8), wherein the computer unit (8) determines a differential pressure between the measured inlet pressure value and the measured outlet pressure value, wherein the installation element (5, 6) is configured as at least one web element, wherein the web element projects into the flow channel with a web element length (LS) which corresponds to at least 25% of a diameter (DS) of the flow channel **characterized in that** a group of web elements extend in a first group plane (11) and a second group plane (12), wherein the first group plane (11) includes a first angle (21) to the longitudinal axis (2) of the tubular element (1) and the second group plane (12) includes a second angle (22) to the longitudinal axis (2) of the tubular element (1).

2. The method of one of the preceding claims, wherein the first group plane (11) intersects with the second group plane (12).

3. The method of one of claims 1 or 2, wherein at least one of the first angle (21) and the second angle (22) measured with respect to the longitudinal axis (2) has a value not equal to 90 degrees.

4. The method of one of the preceding claims, wherein the first group plane (11) and the second group plane (12) each contain at least one web element.

5. The method of one of the preceding claims, wherein a plurality of groups of web elements are arranged one behind the other in the measuring section.

6. The method of one of the preceding claims, wherein the tubular element (1) has an internal diameter, wherein at least one of the web elements has a web element length (LS) which is greater than the internal diameter.

7. The method of one of the preceding claims, wherein at least a part of the web elements is connected to the tubular element in such a way that at least a part of the web element ends is not connected to the tubular element.

8. Use of the inline viscosity measurement method according to one of the preceding claims in a processing method for PVC.

9. The use of claim 8, wherein the processing method for PVC comprises an extruder, wherein the PVC is plasticized by means of the extruder.

10. The use of claim 9, wherein the extruder comprises a counter-rotating twin screw.

11. The use of claim 10, wherein the twin screw has a first twin screw end and a second twin screw end, wherein the flowable medium emerges from the extruder at the second twin screw end.

12. The use of claim 11, wherein a transition piece is arranged between the second twin screw end and the inlet end of the tubular element, wherein the transition piece has a transition piece inlet end and a transition piece outlet end with a corresponding cross-sectional area, wherein the cross-sectional area at the transition piece inlet end differs from the cross-sectional area at the transition piece outlet end.

13. Inline viscosity measuring device (10) for measuring a pressure difference for determining a viscosity of a flowable medium, containing a measuring section which is arranged in a tubular element (1) and which is configured for the flow of the flowable medium, wherein the measuring section is configured as a flow channel, wherein the tubular element (1) comprises a longitudinal axis (2), an inlet end (3) and an outlet end (4), wherein the measuring section extends at least between the inlet end (3) and the outlet end (4), wherein at least one installation element (5, 6) is arranged in the measuring section, wherein an inlet pressure sensor (13) for measuring a measured inlet pressure value is arranged at the inlet end (3), wherein an outlet pressure can be measured at the outlet end (4) so that a measured outlet pressure value is obtainable, wherein the device (10) contains a transducer (7) for converting the measured inlet pressure value and the measured outlet pressure value into measured variables which can be processed by a computer unit (8), so that a differential pressure between the measured inlet pressure value and the measured outlet pressure value can be determined from the measured variables by means of the computer unit (8), wherein the installation element is configured as at least one web element, wherein the web element projects into the flow channel with a web element length (LS) which corresponds to at least 25% of a diameter (DS) of the flow channel, wherein the installation element is configured as at least one group of web elements, wherein the web elements extend in a first group plane (11) and in a second group plane (12), wherein the first group plane (11) includes a first angle (21) with the longitudinal axis of the tubular element and the second group plane (12) includes a second angle (22) with the longitudinal axis (2) of the tubular element (1).

## Revendications

1. Procédé de mesure de viscosité en ligne pour mesurer une différence de pression afin de déterminer la viscosité d'un milieu fluide, dans lequel le milieu fluide s'écoule à travers une section de mesure disposée dans un élément tubulaire (1), la section de mesure étant configurée comme un canal d'écoulement, l'élément tubulaire (1) comportant un axe longitudinal (2), une extrémité d'entrée (3) et une extrémité de sortie (4), la section de mesure s'étendant au moins entre l'extrémité d'entrée (3) et l'extrémité de sortie (4), au moins un élément d'installation (5, 6) étant disposé dans la section de mesure, une pression d'entrée au niveau de l'extrémité d'entrée (3) étant mesurée par un capteur de pression d'entrée (13), de sorte qu'une valeur de pression d'entrée mesurée est obtenue, et une pression de sortie étant mesurée au niveau de l'extrémité de sortie (4) de manière à obtenir une valeur de pression de sortie mesurée, la valeur de pression d'entrée mesurée et la valeur de pression de sortie mesurée étant converties au moyen d'un transducteur (7) en grandeurs mesurées pouvant être traitées par un ordinateur (8), ordinateur (8) déterminant une pression différentielle entre la valeur de pression d'entrée mesurée et la valeur de pression de sortie mesurée, en ce que l'élément d'installation (5, 6) est configuré comme au moins un élément ailette, l'élément ailette faisant saillie dans le canal d'écoulement avec une longueur d'élément ailette (LS) qui correspond à au moins 25 % d'un diamètre (DS) du canal d'écoulement, **caractérisé en ce qu'**un groupe d'éléments ailette s'étend dans un premier plan de groupe (11) et un deuxième plan de groupe (12), le premier plan de groupe (11) formant un premier angle (21) avec l'axe longitudinal (2) de l'élément tubulaire (1) et le second plan de groupe (12) formant un second angle (22) avec l'axe longitudinal (2) de l'élément tubulaire (1).

2. Procédé selon l'une des revendications précédentes, dans lequel le premier plan de groupe (11) coupe le deuxième plan de groupe (12).

3. Procédé selon l'une des revendications 1 ou 2, dans lequel au moins l'un parmi le premier angle (21) et le deuxième angle (22), mesurés par rapport à l'axe longitudinal (2), a une valeur différente de 90 degrés.

4. Procédé selon l'une des revendications précédentes, dans lequel le premier plan de groupe (11) et le deuxième plan de groupe (12) contiennent chacun au moins un élément ailette.

5. Procédé selon l'une des revendications précédentes, dans lequel plusieurs groupes d'éléments ailette sont disposés les uns derrière les autres dans la section de mesure.

6. Procédé selon l'une des revendications précédentes, dans lequel l'élément tubulaire (1) présente un diamètre interne, et dans lequel au moins l'un des éléments ailette présente une longueur d'élément ailette (LS) supérieure au diamètre interne.

7. Procédé selon l'une des revendications précédentes, dans lequel au moins une partie des éléments ailette est reliée à l'élément tubulaire de telle sorte qu'au moins une partie des extrémités des éléments ailette ne soit pas reliée à l'élément tubulaire.

8. Utilisation du procédé de mesure de la viscosité en ligne selon l'une des revendications précédentes dans un procédé de transformation du PVC.

9. Utilisation de la revendication 8, dans laquelle le procédé de traitement du PVC comprend une extrudeuse, le PVC étant plastifié au moyen de cette extrudeuse.

10. Utilisation de la revendication 9, dans laquelle l'extrudeuse comprend une vis jumelée à rotation inverse.

11. Utilisation de la revendication 10, dans laquelle la vis jumelée comporte une première extrémité et une seconde extrémité, le milieu fluide sortant de l'extrudeuse au niveau de la seconde extrémité de la vis jumelée.

12. Utilisation de la revendication 11, dans laquelle une pièce de transition est disposée entre la deuxième extrémité de la vis jumelée et l'extrémité d'entrée de l'élément tubulaire, la pièce de transition comportant une extrémité d'entrée et une extrémité de sortie présentant chacune une section transversale correspondante, la section transversale au niveau de l'extrémité d'entrée de la pièce de transition étant différente de celle au niveau de l'extrémité de sortie de la pièce de transition.

13. Dispositif de mesure de viscosité en ligne (10) destiné à mesurer une différence de pression afin de déterminer la viscosité d'un milieu fluide, comprenant une section de mesure qui est disposée dans un élément tubulaire (1) et qui est configurée pour l'écoulement du milieu fluide, dans lequel la section de mesure est configurée comme un canal d'écoulement, dans lequel l'élément tubulaire (1) comprend un axe longitudinal (2), une extrémité d'entrée (3) et une extrémité de sortie (4), la section de mesure s'étendant au moins entre l'extrémité d'entrée (3) et l'extrémité de sortie (4), au moins un élément d'installation (5, 6) étant disposé dans la section de mesure, un capteur de pression d'entrée (13) destiné à mesurer une valeur de pression d'entrée mesurée étant disposé à l'extrémité d'entrée (3), une pression de sortie pouvant être mesurée à l'extrémité de sortie (4) de sorte qu'une valeur de pression de sortie mesurée puisse être obtenue, dans lequel le dispositif (10) contient un transducteur (7) destiné à convertir la valeur de pression d'entrée mesurée et la valeur de pression de sortie mesurée en grandeurs mesurées pouvant être traitées par un ordinateur (8), de sorte qu'une pression différentielle entre la valeur de pression d'entrée mesurée et la valeur de pression de sortie mesurée puisse être déterminée à partir des grandeurs mesurées au moyen de ordinateur (8), dans lequel l'élément d'installation est configuré sous la forme d'au moins un élément ailette, dans lequel l'élément ailette fait saillie dans le canal d'écoulement avec une longueur ailette (LS) qui correspond à au moins 25 % d'un diamètre (DS) du canal d'écoulement, dans lequel l'élément d'installation est configuré sous la forme d'au moins un groupe d'éléments ailette, dans lequel les éléments ailette s'étendent dans un premier plan de groupe (11) et dans un deuxième plan de groupe (12), le premier plan de groupe (11) formant un premier angle (21) avec l'axe longitudinal de l'élément tubulaire et le second plan de groupe (12) formant un second angle (22) avec l'axe longitudinal (2) de l'élément tubulaire (1).
